# EUROPEAN PATENT APPLICATION

(11) **EP 3 766 473 A1**
(43) Date of publication of application: **20.01.2021**
(21) Application number: 19187137.5
(22) Date of filing: 18.07.2019
(51) Int. Cl.: A61K 8/02, A61K 8/9741, A61K 8/9789, A61Q 19/00, A61Q 19/08

(54) **COSMETIC PREPARATIONS COMPRISING NATURAL ACTIVATORS**

(71) Applicant: ETH Zurich, 8092 Zürich (CH); Universität Basel, 4003 Basel (CH)
(72) Inventor: Detmar, Michael, 8113 Boppelsen (CH); Gousopoulos, Epameinondas, 8037 Zürich (CH); Hamburger, Matthias, 4144 Arlesheim (CH); Potterat, Olivier, 4147 Aesch (CH); Kim, Jihye, 8046 Zürich (CH); Schantl, Antonia Elisabeth, 8052 Zürich (CH)
(74) Representative: Grimm, Siegfried

(57) **Abstract**

The present invention relates to the use of a combination of plant extracts from the genera Salvia, Artemisia, Echinacea, and optionally further plant extracts, for the cosmetic treatment of the skin. The invention further relates to cosmetic preparations which comprise such combination of plant extracts. These plant extracts and cosmetic preparations are particularly useful for stimulation of the lymphatic system, including rejuvenation, anti-ageing, detoxification and increased firmness of the skin, for puffy-eye treatment and / or for anti-swelling effects.

## Description

The present invention relates to cosmetic preparations comprising a combination of plant extracts ("core extract") and to the use of such combination of plant extracts for the cosmetic treatment of the skin. The plant extracts and cosmetic preparations are particularly useful for stimulation of the lymphatic system.

Anti-aging cosmetic preparations with variable effects are widely known. Also cosmetic preparations comprising plant extracts which are aimed at skin treatment are known per se and well investigated. In the past, there have already been various compositions in cosmetics and in dermatology whose task was to protect skin, in particular human skin. For example, EP18211306.8 (unpublished) relates to the use of plant extracts from the genus Daphne for the cosmetic treatment of the skin and to cosmetic preparations comprising such plant extracts. According to this document, the plant extracts and cosmetic preparations are particularly useful for stimulation of the lymphatic system.

Nevertheless, there continues to be a need to improve or to modify skin protection because the environmental conditions and also living conditions and habits of users are changing. It is further believed there is an ever-existing need to provide alternatives to present treatments, particularly to meet with consumers preferences, to meet with various types of skin and / or to comply with legal provisions.

In consequence, there is a need for further cosmetic compositions, particularly for improved cosmetic compositions, to stimulate the lymphatic system.

Thus, it is an object of the present invention to mitigate at least some of these drawbacks of the state of the art. In particular, it is an aim of the present invention to provide new combined plant extracts and new preparations adapted to such uses.

One or more of these objectives are achieved by the compositions as defined in claim 1. Further aspects of the invention are disclosed in the specification and independent claims, preferred embodiments are disclosed in the specification and the dependent claims.

The present invention will be described in more detail below. It is understood that the various embodiments, preferences and ranges as provided / disclosed in this specification may be combined at will. Further, depending of the specific embodiment, selected definitions, embodiments or ranges may not apply.

Unless otherwise stated, the following **definitions** shall apply in this specification:
As used herein, the term "a", "an", "the" and similar terms used in the context of the present invention (especially in the context of the claims) are to be construed to cover both the singular and plural unless otherwise indicated herein or clearly contradicted by the context.

As used herein, the terms "including", "containing" and "comprising" are used herein in their open, non-limiting sense. The term "containing" shall include "comprising", "essentially consisting of" and "consisting of".

The term "treatment" shall include the prevention, inhibition and delay of progression of negative effects and / or the promotion of desirable effects.

The present invention will be better understood by reference to the **figures;**

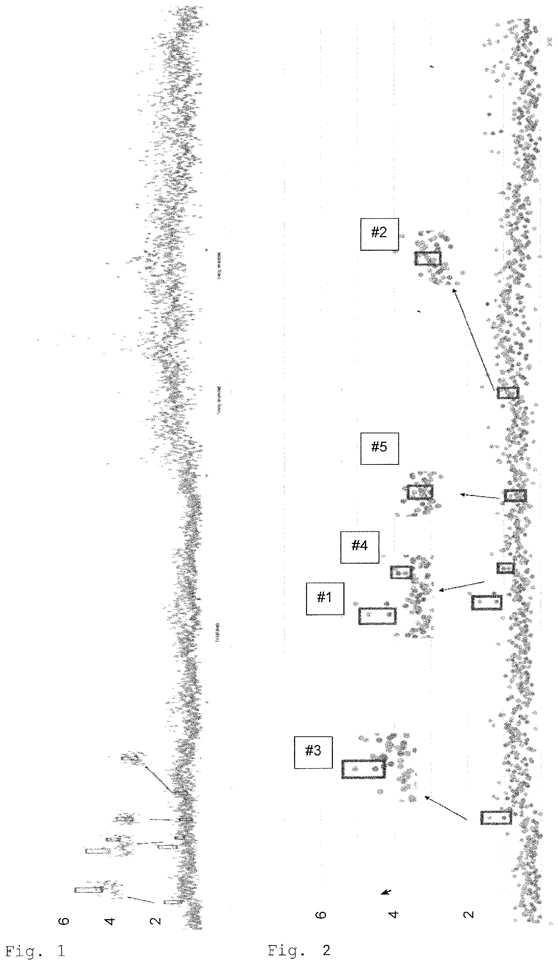

Fig. 1 shows results of a library screening of approx. 2050 plant extracts as outlined in example 1. x-axis: # of plant extracts; y-axis: sprouts / bead.
Fig. 2 shows an enlarged section of fig. 1. x-axis: # of plant extracts; y-axis: sprouts / bead. Marked and enlarged for clarity reasons appear the following extracts (from left to right): Echinacea angustifolia (#3, narrow leaved coneflower), Salvia hispanica (#1, Chia), Equisetum arvense (#4, Horsetail herb), Artemisia abrotanum (#5, Southernwood herb), Artemisia vulgaris (#2, Mugwort)

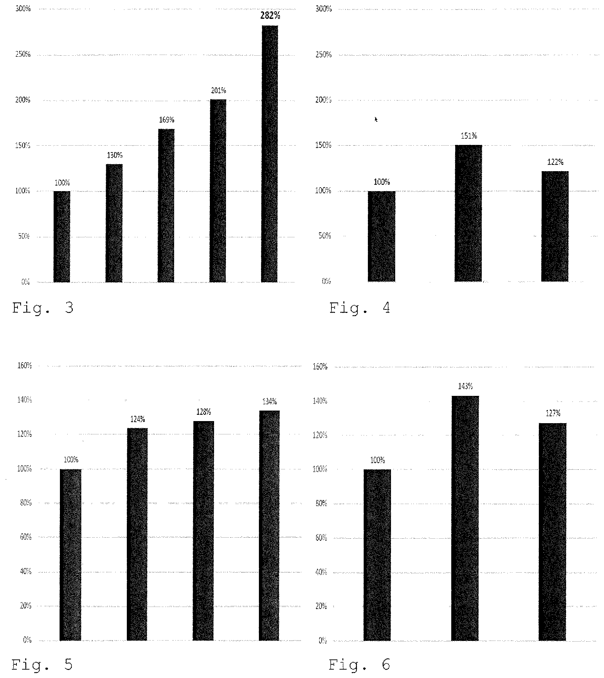

Fig. 3 shows activity of the core extract ingredients in spouting assay as outlined in example 2. x-axis from left to right: Glycerol (control - respective to the extract concentration) - Salvia hispanica/Chia (1%) - Artemisia vulgaris/ Mugwort herb (0.05%) - Echinacea angustifolia / Narrow-leaved coneflower (0.05%) - Inventive combination (Chia (1%), Mugwort herb (0.05%), Narrow-leaved coneflower (0.05%)); y-axis: percent (%) activity.
Fig. 4 shows activity of Equisetum arvense / Horsetail herb and Artemisia abrotanum / Southernwood herb in spouting assay as outlined in example 2. x-axis from left to right: Glycerol. (control - respective to the extract concentration) - Equisetum arvense/Horsetail herb (0.1%) - Artemisia abrotanum / Southernwood herb (0.05%); y-axis: percent (%) activity.
Fig. 5 shows activity of the core extract ingredients in lymphatic regeneration/migration assay as outlined in example 3. x-axis from left to right: Glycerol (control - respective to the extract concentration) - Salvia hispanica (Chia; 1%) - Artemisia vulgaris (Mugwort herb); 0.05%) - Echinacea angustifolia (Narrow-leaved coneflower; 0.05%); y-axis: percent (%) activity.
Fig. 6 shows activity of Equisetum arvense (Horsetail herb) and Artemisia abrotanum (Southernwood herb) in lymphatic regeneration / migration assay as outlined in example 6. x-axis from left to right: Glycerol (control - respective to the extract concentration) - Horsetail herb (0.1%) - Southernwood herb (0.05%); y-axis: percent (%) activity.

In more general terms, in a **first aspect,** the invention relates to the use of a combination of specific plant extracts as described herein for the cosmetic treatment of the skin. It was surprisingly found that such extract combination stimulates the lymphatic system and thus provides for a number of beneficial cosmetic applications.

This aspect of the invention shall be explained in further detail below:
**Use:** As discussed herein, the invention is directed to the use of a combination of specific plant extracts as discussed herein for the cosmetic treatment of the skin. In an embodiment, such use excludes the therapeutic treatment of the skin.

**Combination of specific plant extracts:** It was found that extracts of
#1 genus Salvia, particularly the species Salvia hispanica, in combination with
#2 genus Artemisia, particularly species Artemisia vulgaris, and in combination with
#3 genus Echinacea, particularly the species Echinacea angustifolia,
possess particularly pronounced lymphangiogenic properties. Herein, the combination of these 3 plant extracts is referred-to as core-extract.

It was further found that the core extract may be complemented by one or more of the following extracts:
#4 genus Equisetum, particularly the species Equisetum arvense, and
#5 genus Artemisia, particularly the species Artemisia abrotanum (#5) to thereby further enhance lymphangiogenic properties and / or to reduce undesired side-effects and / or to provide additional beneficial effects to the skin.

Plant extracts #1 - #5 are known per se. However, the combination of #1 with #2 and #3 (i.e. the core extract) is not yet described in literature. Its particular beneficial properties are unexpected and outlined herein.

In advantageous embodiments, extracts comprise:
- plant extracts of #1, #2, #3 and #4; or
- plant extracts of #1, #2, #3 and #5; or
- plant extracts of #1, #2, #3 and #4 and #5.

Suitable extracts may be identified using a botanical extract library and a high-throughput in vitro 3D screening assay. The extracts were found to induce sprouting and migration of cultured human lymphatic endothelial cells. These results suggest a novel role of these extracts in promoting lymphatic vascular activity and function.

**Plants from the genus Salvia** (#1): This genus is well-known and includes the species Salvia hispanica (Chia). Plants from this genus contain characteristic diterpenes, such as carnosol.

**Plants from the genus Artemisia** (#2): This genus is well-known and includes species Artemisia vulgaris (mugwort herb). Plants from this genus contain characteristic Sesquiterpene lactones, such as vulgarin.

**Plants from the genus Echinacea** (#3): This genus is well-known and includes the species Echinacea angustifolia (narrow-leaved coneflower). Plants from this genus contain characteristic alkylamides, such as dodecatetraenoic acid isobutylamide.

**Plants from the genus Equisetum** (#4): This genus is well-known and includes the species Equisetum arvense (horsetail herb). Plants from this genus contain characteristic flavonoids, such as isoquercitrin.

**Further Plants from the genus Artemisia** (#5): This genus is well-known and also includes the species Artemisia abrotanum (Southernwood herb). Plants from this genus contain characteristic sesquiterpene lactones.

**Plant extracts:** Preparing plant extracts is well-known in the field. Known technologies may be applied to obtain plant extracts from plants of the genera described herein. Plant extracts are obtainable by using a polar solvent or mixture of polar solvents. Suitable polar solvents include: C1-C4 alcohols (including methanol, ethanol, propanol, and butanol), C1-C4 diols ("glycols"; including ethylene-, propylene-, butylene-glycol), glycerol, acetone, ethyl acetate). Such polar solvents may be combined with water. Further, plant extracts are obtainable by using an apolar solvent. Suitable apolar solvents include: C4-C10 alkanes (including (cyclo)-hexane), C1-C2 alkylhalogenides (including methylene chloride, chloroform), C1-C4 dialkylethers (including diethylether, t-butylmethylether). Still further, plant extracts may be obtained by using supercritical fluids (particularly CO₂); and/ or mixtures of CO₂ with C1-C3 alcohols (particularly: methanol, ethanol, propanol) or with acetone.

Fresh or dried extracts from the plant's root may be used. Further, fresh or dried extracts from the plant's green part may be used. When using the green part, extracts may stem from the green part as a whole or from one or more of flowers, fruits, leaves, stems and stalks.

The term "plant extracts" shall include (i) crude extracts obtained from the plant or parts thereof and (ii) extracts enriched in either of diterpenes, sesquiterpene lactones, alkylamides, obtained from the plant or parts thereof, and (iii) purified substances therefrom, such as carriosol, vulgarin, dodecatetraenoic acid isobutylamide.

**Stimulation of the lymphatic system:** Lymph vessels fulfill an important role in order to maintain the microenvironment surrounding tissue in a constant state by removing unwanted substances present in skin as well as water and protein constantly escaping from blood vessels. It was found that plant extracts as described herein activate the lymphatic system, particularly the lymphatic vasculature. This action was tested using in vitro test methods. In particular, using a model of sprouting assay developed previously (SCHULZ MM ET AL., PNAS 2012: PMID 22949700), a library of plant extracts was screened for extracts inducing lymphatic endothelial cell (LEC) sprouting. The key findings were further verified and analysed to characterize the active ingredients. The advantageous effects of the plant extracts were further confirmed by conducting relevant in vitro migration assays. Given the positive results in all above mentioned assays, one can deduct the positive effect of the plant extracts and the active ingredient thereof, on improving lymphatic vascular function.

The invention thus relates to the use of plant extracts as described herein for promoting activation of the lymphatic system, particularly for stimulating the lymphatic vasculature.

The above findings are further and subsequently linked to skin detoxification and anti-aging properties, as key contributions of the improved function of the lymphatic vasculature. Accordingly, the cosmetic treatment provides for a number of desirable effects. In an advantageous embodiment, the cosmetic treatment provides for rejuvenation of the skin. In a further advantageous embodiment, the cosmetic treatment provides for an anti-aging effect of the skin. In a further advantageous embodiment, the cosmetic treatment includes puffy-eye treatment. In a further advantageous embodiment, the cosmetic treatment provides for detoxification of the skin. In a further advantageous embodiment, the cosmetic treatment increases firmness of the skin. In a further advantageous embodiment, the cosmetic treatment provides for an anti-swelling effect.

The plant extracts described herein may be used in a broad concentration range to achieve the desirable effects described. A suitable range may be determined by routine experiments, considering the type of cosmetic composition and the effect desired. Useful concentration ranges depend on the type of plant extract to be used. When using crude extracts obtained from the plant or parts thereof, suitable concentration are in the range of 0.0001 - 20 wt%, such as 0.001 - 10 wt%. When using extracts enriched in its active ingredient, obtained from the plant or parts thereof, suitable concentrations are in the range of 0.00001 - 10 wt%, such as 0.0001 - 5 wt%. When using purified substances therefrom, suitable concentrations are in the range of 10 pM - 10 mM, such as 1 nM - 1 mM.

In a **second aspect,** the invention relates to cosmetic preparations comprising the core extract as described herein.

Given the key role of the lymphatic system in the maintenance of skin homeostasis and aging, one aspect of the invention comprises the provision of cosmetic products. Without being bound to theory, it is believed that such cosmetic product will exert its action through the activation of the lymphatic vasculature, increasing the uptake of extravasated fluid, cellular or molecular waste, thus supporting skin detoxification (detox) and function, also bearing anti-aging properties.

This aspect of the invention shall be explained in further detail below:
As discussed above, such core extracts contain diterpenes and sesquiterpene lactones and alkylamides. As discussed above, these plant extracts were found particularly useful in cosmetic compositions for skin treatment.

**Cosmetic preparation:** Cosmetic preparations are known per se. However, until now no such preparation comprising plant extracts from members of #1 in combination with #2 and #3 were described. The invention therefore provides for such cosmetic preparations. Accordingly, such preparations comprise plant extracts containing diterpenes, sesquiterpene lactones and alkylamides.

The skilled person is in a position to formulate cosmetic preparations containing such extracts. Suitable amounts of plant extract within the cosmetic preparation may vary over a broad range and depend (inter alia) on the type of plant extract used in the cosmetic preparation. Suitable ranges are disclosed above and include, for example, 0.0001 - 20 wt% in case of crude plant extracts; 0.00001 - 10 wt% in case of enriched extracts; 10 pM - 10 mM in case of purified substances of said plant extracts within said cosmetic preparation. The indicated range is broad, due to the large variety of plant extracts suitable and due to the large spectrum of possible preparations. Suitable plant extracts may be obtained as discussed above, first aspect of the invention.

**Formulations:** Cosmetic preparations may be provided in any conventional form known to the skilled person. In one embodiment, the cosmetic preparation is in the form of a liquid formulation. Such liquid formulations include gels, lotions, milks, emulsions, a spray, an oil and foams. In one further embodiment, the cosmetic preparation is in the form as a semisolid formulation or as a solid formulation. Such formulations include creams, ointments, powders and sticks. These types of formulations are known per se.

In a further aspect, the invention provides for a skin detoxification ameliorant comprising the core plant extracts described herein and for a skin anti-aging ameliorant comprising core plant extracts described herein.

**Excipients:** Cosmetic preparations typically comprise excipients. Such excipients are known per se and the skilled person is in a position to select them in view of the intended form of application for the plant extracts described herein. Accordingly, the invention provides for a cosmetic preparation comprising plant extracts as described herein and further comprising one or more cosmetic excipients; said excipients preferably being selected from the group consisting of emollients, moisturizers, thickeners, emulsifiers, colorings, detergents, disinfectants, antioxidants, buffers, matting agents, exfoliating agents, aromas, essential oils, vitamins, and UV filters.

**Extracellular matrix components:** It was further found that extracellular matrix components beneficially improve or support the cosmetic effects obtained when using the core extracts described herein. Particularly, a positive synergistic effect is observed when providing cosmetic compositions as described herein further containing extracellular matrix components. Such extracellular matrix components are known to the skilled person and include collagen, elastin, and hyaluronic acid; particularly hyaluronic acid. Accordingly, the invention also provides for the use of core plant extracts as described herein in combination with extracellular matrix components for the cosmetic treatment of the skin. Further, the invention provides for cosmetic preparations for skin treatment comprising core extracts and extracellular matrix components, both as described herein.

In a **third aspect,** the invention relates to a method of cosmetically treating the skin. This method comprises the step of applying to skin in need thereof a preparation as described herein, second aspect of the invention. This aspect shall be described in further detail below:
Cosmetic treatment: It is to be understood that cosmetic treatment excludes therapeutic treatments and therefore does not require supervision by a physician. Advantageously, the skin is treated to stimulate the lymphatic system.

The aforementioned core extracts are extremely useful for supporting the function of lymph vessels. Symptoms accompanying lymph vessel dysfunction are discussed herein. The core extracts are effective for treating the lymph vessels, particularly for stimulating the lymphatic vasculature.

As discussed above, the stimulation of the lymphatic system provides for a number of desirable effects. Accordingly, the invention provides for a method of cosmetically treating the skin to effect one or more of the following: rejuvenation of the skin; anti-aging of the skin; puffy-eye treatment; detoxification of the skin; firmness of the skin and anti-swelling effect.

Still further, the inventive treatment protects the skin. Still further, the inventive treatment counteracts swelling of the skin. Still further, the inventive treatment increases the longevity of skin cells. Still further, the inventive treatment counteracts intrinsic and/or extrinsic skin ageing.

As a consequence, the invention provides for a cosmetic method for treating the skin, said method comprises the step of applying a composition as described herein to the skin of a subject requiring activation of lymph vessel function. Said treatment particularly includes the cosmetic treatments discussed above.

To further illustrate the invention, the following **examples** are provided. These examples are provided with no intent to limit the scope of the invention.

### Example 1: Library screening of 1954 plant extracts

After a 24 hour incubation of fluorescently labeled LEC (lymphatic endothelial cell)-covered dextran beads with 10 µg/mL of extracts from a library of 1954 plant-derived samples, sprout formation was assessed by determining the ratio of sprouts per bead. 0.1 % DMSO served as a vehicle control. Each extract was tested as 1-plicate and the entire library was screened in two independent replicates. Fig. 1 shows the results obtained for replicates 1 and 2. Fig. 2 is an enlarged section of fig. 1. It shows the results obtained for replicates 1 and 2; enlarged are:
- Echinacea angustifolia (#3): 1.0 and 1.4 sprouts/bead
- Salvia hispanica (#1) 1.2 and 1.7 sprouts/bead
- Equisetum arvense (#4): 1.0 and 0.9 sprouts/bead
- Artemisia abrotanum (#5): 0.57 and 0.85 sprouts / bead
- Artemisia vulgaris (#2): 0.86 and 0.75 sprouts / bead
The results indicate that various species and various extracts are suitable for the uses and applications described herein.

**Example 2: LEC sprouting of** Echinacea angustifolia (#3), Salvia hispanica (#1), Equisetum arvense (#4), Artemisia abrotanum (#5), Artemisia vulgaris (#2) **extracts:**
The Echinacea angustifolia (narrow leaved coneflower), Salvia hispanica (Chia), Equisetum arvense (Horsetail herb), Artemisia abrotanum (Southernwood herb), Artemisia vulgaris (Mugwort) extracts purchased from commercial sources induced LEC sprouting at concentrations ranging from 0.05%-1%. Sprout formation is expressed as percent activity and was assessed by determining the ratio of sprouts per bead in comparison to respective % glycerol (negative control) . Each sample was tested as 6-replicates, results are shown in **fig. 3 and 4****.**

Fig. 3 The *Chia, Mugwort Herb and Narrow-Leaved Coneflower* (from second left to right) extracts potently induced LEC sprouting at concentrations of 1%, 0.05% and 0.05% respectively. The inventive mixture of all three extracts revealed an additive effect, increasing the overall activation of the LECs.

Fig. 4 The *Horsetail Herb and Southernwood* extracts potently induced LEC sprouting at concentrations of 0.1%.

**Example 3: LEC migration upon treatment with** Echinacea angustifolia (narrow-leaved coneflower), Salvia hispanica (Chia), Equisetum arvense (Horsetail herb), Artemisia abrotanum (Southernwood herb), Artemisia vulgaris (Mugwort) **extracts:**
LEC migration was increased by the *Chia, Mugwort Herb and Narrow-Leaved Coneflower as well as the Southernwood and Horsetail* extracts in an *in vitro* lymphatic regeneration/migration assay. The quantification was presented as % open wound area at 3 h in comparison to 0 h (100 %) open wound area. The results are representative for 2 independent experiments, each with a minimum of 6 replicates. The results are shown in **fig. 5 and 6****.**

## Claims

1. A cosmetic preparation, particularly for skin treatment, wherein the preparation comprises:
• plant extracts from members of the genus Salvia (#1), particularly the species Salvia hispanica, and
• plant extracts from members of the genus Artemisia (#2), particularly species Artemisia vulgaris, and
• plant extracts from members of the genus Echinacea (#3), particularly the species Echinacea angustifolia.

2. The cosmetic preparation according to claim 1, wherein the preparation additionally comprises:
• plant extracts from members of the genus Equisetum (#4), particularly the species Equisetum arvense, and / or
• further plant extracts from other members of the genus Artemisia (#5), particularly the species Artemisia abrotanum.

3. The cosmetic preparation of claim 1 or 2, wherein each of said plant extracts are present in an amount
• of 0.0001 - 20 wt% in case of crude plant extracts,
• of 0.00001 - 10 wt% in case of enriched extracts,
• of 10 pM - 10 mM in case of purified substances of said plant extracts,
based on the total weight of the preparation.

4. The cosmetic preparation according to any of claims 1 - 3, further comprising one or more cosmetic excipients selected from the group consisting of emollients, moisturizers, thickeners, emulsifiers, colourings, detergents, disinfectants, antioxidants, buffers, matting agents, exfoliating agents, aromas, essential oils, vitamins, UV filters, and extracellular matrix components (particularly collagen, elastin, and hyaluronic acid).

5. The cosmetic preparation according to any of claims 1 - 4, formulated as
• a liquid formulation (preferably a gel, a lotion, a milk, an emulsion, a foam, a spray, an oil); or
• a semisolid or solid formulation (preferably a cream, an ointment, a stick, a powder);
• a shaped article (preferably a tissue mask or a facial mask) further comprising a supporting material.

6. The cosmetic preparation according to any of claims 1 - 5, where said plant extracts are
• extracts from the plant's root (particularly bark root); and/or
• from the plant's green part (particularly flowers, fruits, leaves, stems and stalks);
both fresh and dried.

7. The cosmetic preparation according to any of claims 1 - 3, where said plant extracts are obtained
• by using a polar solvent, preferably selected from the group of glycerol, C1-C4 alcohols, C1-C4 diols, acetone, ethyl acetate in each case optionally in the presence of water (preferably glycerol);
• by using an apolar solvent (preferably selected from C4-C10 alkanes, C1-C2 alkylhalogenides, C1-C4 dialkylethers); and / or
• by using supercritical fluids (preferably CO₂; and / or mixtures of CO₂ with C1-C3 alcohols or with acetone).

8. A method of cosmetically treating the skin, particularly cosmetically and non-therapeutically treating the skin, wherein the method comprises applying to skin in need thereof a cosmetic preparation according to any of claims 1 - 7.

9. The method of claim 8, wherein skin is treated to stimulate the lymphatic system.

10. The method according to any of claims 8 - 9, wherein the skin is treated for
• rejuvenation of the skin,
• anti-aging of the skin,
• puffy-eye treatment,
• detoxification of the skin,
• increased firmness of the skin, and / or
• anti-swelling effect.

11. The method according to any of claims 8 - 10, where said treatment
• increases the longevity of skin cells;
• counteracts intrinsic and / or extrinsic skin ageing;
• protects the skin; and / or
• counteracts swelling of the skin.

12. Use of a combination of plant extracts for the cosmetic treatment, particularly the non-therapeutic cosmetic treatment, of the skin,
• wherein said plant extracts are selected from the group consisting of #1, #2, and #3 as defined in claim 1 and optionally #4 and / or #5 as defined in claim 2; and
• wherein said cosmetic treatment involves stimulation of the lymphatic system.

13. The use according to claim 12, where said cosmetic treatment is selected from:
• rejuvenation of the skin,
• anti-aging of the skin,
• puffy-eye treatment,
• detoxification of the skin,
• increased firmness of the skin, and
• anti-swelling effect.

14. The use according to any of claims 12 - 13, where each of said plant extracts is used in a concentration between
• 0.0001 - 20 wt% in case of crude plant extracts,
• 0.00001 - 10 wt% in case of enriched extracts,
• 10 pM - 10 mM in case of purified substances of said plant extracts.
